# FASCICULE DE BREVET EUROPEEN

(11) **EP 3 664 727 B1**
(45) Date de publication et mention de la délivrance du brevet: **18.08.2021**
(21) Numéro de dépôt: 18762013.3
(22) Date de dépôt: 07.08.2018
(51) Int. Cl.: A61B 17/20

(54) **DISPOSITIF D'INCISION SUPERFICIELLE, NOTAMMENT POUR DIAGNOSTIC ALLERGIQUE**
OBERFLÄCHENSCHNEIDVORRICHTUNG, INSBESONDERE FÜR ALLERGIEDIAGNOSTIK
SURFACE CUTTING DEVICE, IN PARTICULAR FOR ALLERGY DIAGNOSIS

(30) Priorité: 08.08.2017 FR 1757581
(43) Date de publication de la demande: 17.06.2020
(73) Titulaire: A2M, 85700 Saint Mesmin (FR)
(72) Inventeur: GROZELIER, Isabelle, 85700 Saint Mesmin (FR)
(74) Mandataire: Regimbeau
(86) Numéro de dépôt international: PCT/EP2018/071426
(87) Numéro de publication internationale: WO 2019/030238

(56) Documents cités:
- US-A- 3 194 237
- US-A- 4 583 982
- US-A1- 2007 016 100
- US-A1- 2016 256 636

## Description

### DOMAINE TECHNIQUE

La présente invention concerne le domaine des dispositifs d'incision superficielle de la peau d'un patient, notamment pour la réalisation d'un diagnostic allergique.

Les tests de diagnostics allergiques cutanés sont généralement dénommés « prick test ».

### ETAT DE L'ART

Les tests de diagnostics allergiques sont des examens médicaux consistant à faire pénétrer superficiellement quelques micro-gouttes d'un produit (allergène par exemple) dans la partie supérieure de l'épiderme d'un patient, par exemple son avant-bras, afin de tester la réactivité de ce patient audit produit.

En pratique, après avoir déposé une goutte de produit sur la peau, on pique la peau à travers la goutte avec une pointe, pour faire pénétrer du produit sous la peau, puis on attend quinze à vingt minutes avant d'interpréter la réaction cutanée.

Plusieurs systèmes existent actuellement sur le marché pour pratiquer l'incision de la peau afin d'assurer la pénétration de produit sous la peau. La structure générale des systèmes connus est quasiment toujours la même : un bâtonnet en plastique ou en métal est équipé d'une, voire deux ou plusieurs, pointe(s) d'incision à son extrémité.

On trouvera notamment des exemples de tels dispositifs connus dans les documents FR 1292190, FR 1309352, FR 1349909, FR 2119120, FR 2474856, FR 2535602 et FR 2748647, ainsi que dans les documents US 2522309, US 3136314, US 3221739, US 3596660, US 3675766, US 2016/256636, US 2007/016100, US 3194237 et US 4583982.

### PROBLEME POSE

Les dispositifs jusqu'ici proposés ont déjà rendu de grands services. Cependant, ils ne donnent pas totalement satisfaction.

En particulier les systèmes actuellement connus ne garantissent pas de faire pénétrer une quantité optimale et reproductible de produit dans l'épiderme et des réactions faussement négatives existent de ce fait sans qu'elles soient réellement identifiées.

De plus la quantité utile de produit consommé pour la réalisation de chaque incision et donc chaque test, est très largement supérieure à celle qui est nécessaire.

Les pointes d'incision actuellement disponibles nécessitent également un geste précis et reproductible. Une pression trop forte de l'opérateur peut en effet déclencher un saignement ou un résultat faussement positif. Inversement une pression trop faible peut donner un test faussement négatif.

Par ailleurs l'utilisation actuelle d'une très large majorité des pointes d'incision connues implique un contact du distributeur de produit avec la peau, le produit restant dans le distributeur est ensuite utilisé pour d'autres patients ce qui implique potentiellement un risque de contamination entre les différents patients.

### BASE DE L'INVENTION

Le but principal de l'invention est ainsi de proposer un dispositif d'incision perfectionné par rapport à l'état de la technique.

Un but de l'invention est notamment de proposer une pointe perfectionnée adaptée pour introduire du produit en volume contrôlé dans l'épiderme et ce à une profondeur contrôlée avec précision et reproductible, par un geste simple.

Un autre but de l'invention est de proposer un dispositif d'incision économique qui garantit, sans que des précautions particulières ne soient nécessaires, une incision strictement limitée au minimum nécessaire pour optimiser un test de diagnostic allergique.

Les buts précités sont atteints dans le cadre de l'invention grâce à un dispositif d'incision comprenant :
- un élément de préhension manuelle et
- un ensemble d'incision placé sur une extrémité de l'élément de préhension et comprenant un poinçon scindé en au moins deux pointes d'incision,
caractérisé en ce que l'une au moins de ces pointes est articulée à déplacement sur l'élément de préhension pour être mobile entre une position de repos et d'incision dans laquelle les pointes sont adjacentes et une position de travail et d'injection dans laquelle les pointes sont écartées, et que le dispositif comprend des moyens de sollicitation adaptés pour déplacer chaque pointe mobile en position de travail, lorsque ces moyens de sollicitation viennent en appui sur la peau.

D'autre exemples de l'invention sont définis dans les revendications dépendantes.

### DESCRIPTION DETAILLEE DES FIGURES

D'autres caractéristiques, buts et avantages de l'invention apparaîtront à la lecture de la description détaillée qui va suivre, et en regard des dessins annexés donnés à titre d'exemple non limitatif et sur lesquels :
- la figure 1 représente une première vue latérale d'ensemble d'un dispositif d'incision conforme à la présente invention,
- la figure 2 représente une vue de détail partiel à échelle agrandie de l'extrémité d'un dispositif d'incision conforme à l'invention,
- la figure 3 représente une deuxième vue latérale d'ensemble d'un dispositif d'incision conforme à l'invention selon un angle d'observation orthogonal à la figure 1,
- les figures 4, 5 et 6 représentent le même dispositif conforme à l'invention à trois étapes successives d'utilisation pour une incision cutanée,
- la figure 7 représente schématiquement une vue en coupe de l'épiderme dans lequel une injection a été réalisée grâce à un dispositif d'incision conforme à l'invention, et
- les figures 8 et 9 représentent deux vues latérales similaires à la figure 1 de variantes d'un dispositif d'incision conforme à la présente invention.

### DESCRIPTION DETAILLEE DE L'INVENTION

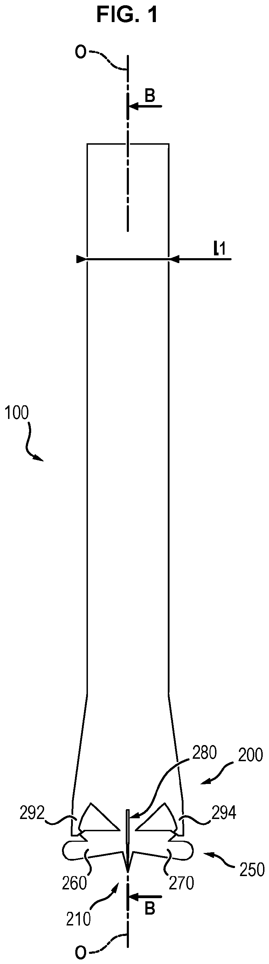

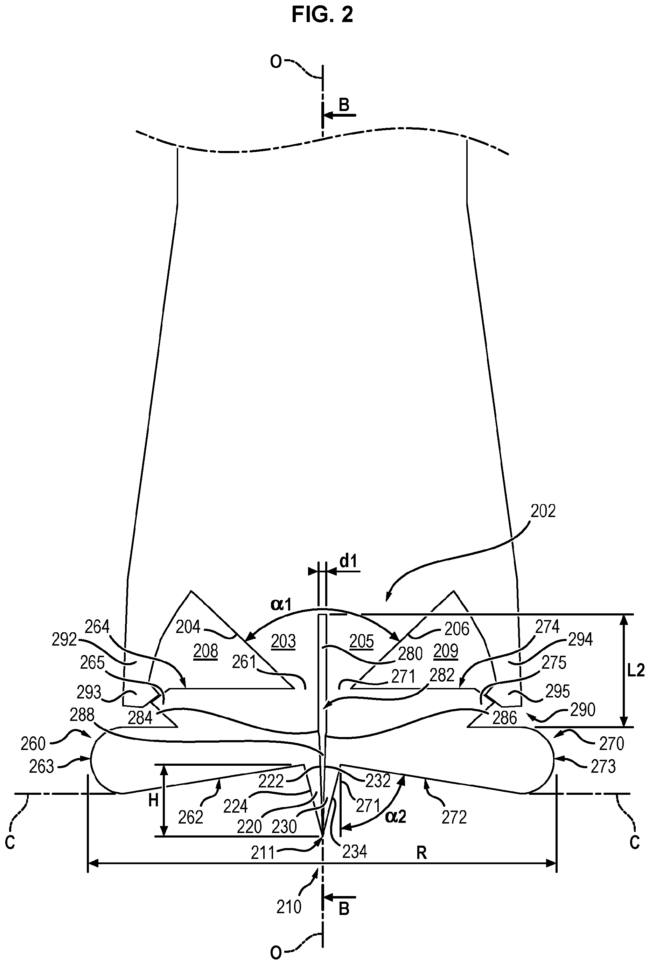

Comme indiqué précédemment, et comme on le voit sur les figures 1, 2 et 3, le dispositif d'incision superficielle conforme à la présente invention comprend un élément allongé de préhension manuelle 100 et un ensemble d'incision 200 placé sur une extrémité de l'élément de préhension 100.

L'ensemble d'incision 200 comporte un poinçon 210 et des moyens de sollicitation 250.

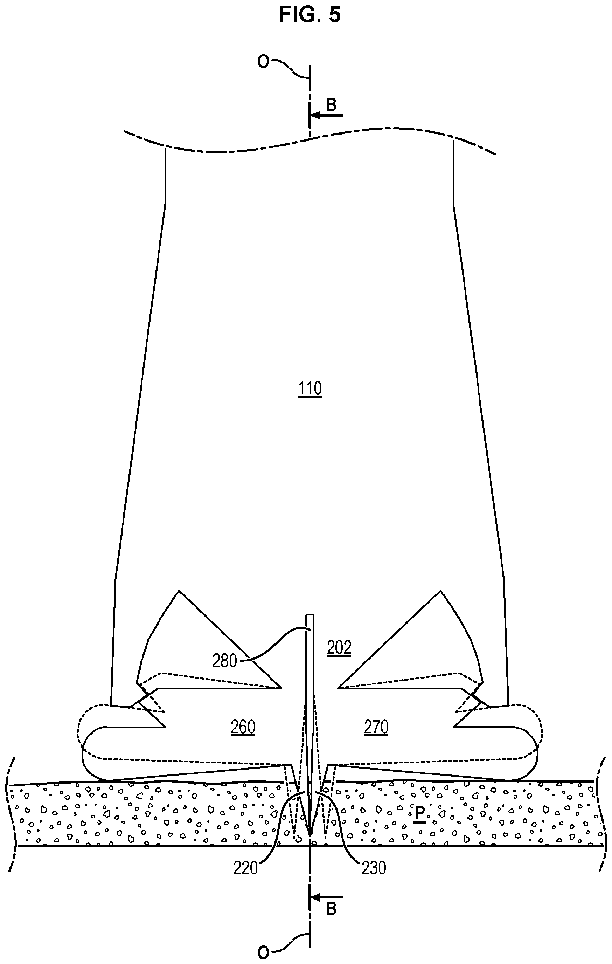

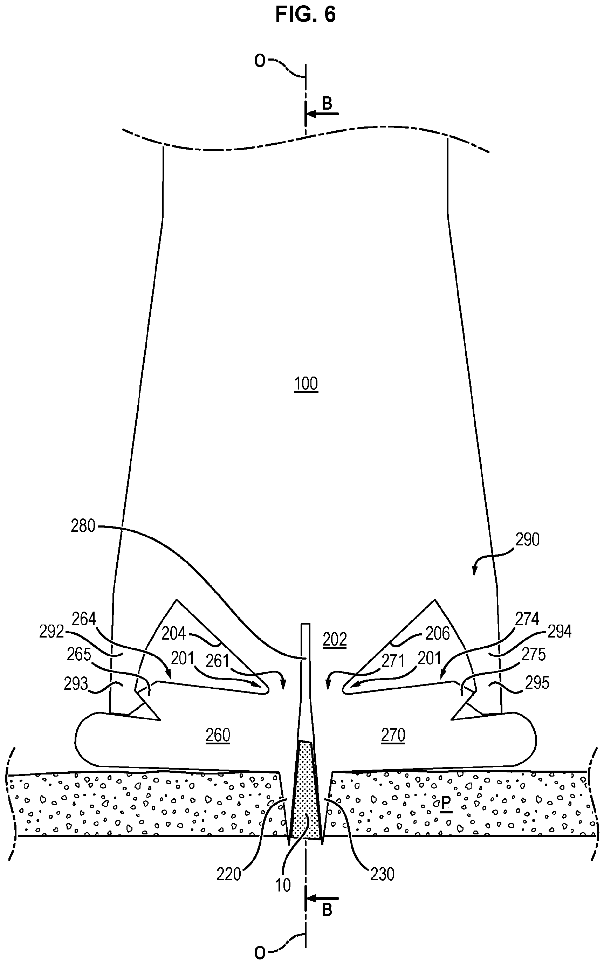

Le poinçon 210 est scindé en au moins deux pointes ou aiguilles 220, 230 d'incision. L'une au moins de ces deux pointes 220, 230 est articulée à déplacement sur l'élément de préhension 100 pour être mobile entre une position de repos et d'incision illustrée sur les figures 1, 2 et 4 et une position de travail et d'injection illustrée sur les figures 5 et 6.

En position de repos, les deux pointes 220, 230 sont adjacentes et prêtes pour une incision en un point quasiment unique.

En position de travail et d'injection, après avoir pénétré superficiellement la peau, les deux pointes 220, 230 sont écartées.

Les moyens de sollicitation 250 sont adaptés pour déplacer la pointe mobile 220 et/ou 230 en position de travail lorsqu'ils viennent en appui sur la peau d'un individu.

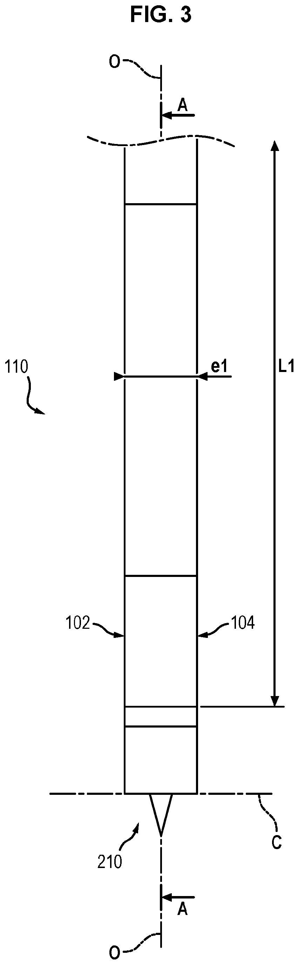

Le dispositif conforme à l'invention présente de préférence un axe de symétrie longitudinal O-O. Plus précisément encore, le dispositif d'incision conforme à l'invention présente de préférence deux plans de symétrie A-A et B-B représentés notamment sur les figures 1 et 3, sécants selon l'axe O-O.

La vue latérale de la figure 1 est parallèle au plan de symétrie A-A et orthogonale au plan de symétrie B-B. La vue illustrée sur la figure 3 est quant à elle parallèle au plan de symétrie B-B et orthogonale au plan de symétrie A-A.

Dans le cadre de l'invention, de préférence le poinçon 210 est scindé en deux pointes d'incision 220, 230 et dans ce mode de réalisation préférentiel chacune des deux pointes 220, 230 est articulée à rotation sur l'élément de préhension 100 entre la position de repos et d'incision dans laquelle les deux pointes sont adjacentes et la position de travail et d'injection dans laquelle les deux pointes sont écartées, par rotation autour d'un axe respectif orthogonal au plan A-A et parallèle au plan B-B.

L'élément de préhension manuelle 100 peut faire l'objet de nombreux modes de réalisation.

De préférence, l'élément de préhension manuelle 100 est formé d'un manche allongé centré sur l'axe O-O.

La géométrie de la section de l'élément de préhension manuelle 100, dans un plan orthogonal à l'axe O-O, peut également faire l'objet de nombreuses variantes de réalisation.

Cette section peut par exemple être circulaire de révolution autour de l'axe O-O ou en forme de polygone, notamment de quadrilatère, par exemple rectangulaire ou carrée.

L'élément de préhension manuelle 100 peut avoir par exemple, sans que ceci ne soit limitatif, une longueur minimale L1 selon l'axe O-O de l'ordre de 45mm, et une section rectangulaire ayant une largeur l1 selon le plan A-A et donc orthogonalement au plan B-B comprise entre 4 et 15 mm et une épaisseur e1 selon le plan B-B et donc orthogonalement au plan A-A comprise entre 0,2 (si métallique par exemple) et 5mm.

Comme on le voit sur les figures annexées, l'extrémité de l'élément de préhension manuelle 100 qui porte l'ensemble d'incision 200 a de préférence une forme effilée, par exemple sous la forme d'un cône 202 convexe vers l'extérieur du dispositif ou d'un coin d'octaèdre. L'angle au sommet α1 du cône 202 est typiquement de l'ordre de 90°.

Les moyens de sollicitation 250 présentent de préférence deux languettes 260, 270 diamétralement opposées par rapport au plan B-B. Les languettes 260, 270 sont de préférence venues de matière au voisinage de la pointe du cône 202. Les languettes 260, 270 s'étendent chacune dans une direction orthogonale à l'axe longitudinal O-O et plus précisément au plan B-B. La géométrie de la forme des moyens de sollicitation 250 pourra être optimisée pour l'amélioration de sa fonction d'appui sur la peau, par exemple par un élargissement à son extrémité.

Comme on le verra ultérieurement, le cône 202 est muni d'une fente axiale 282 dans le plan A-A, destinée à former un réservoir de produit 280.

Dans le cadre des schémas illustratifs, les deux languettes 260, 270 sont ainsi articulées sur le sommet des deux segments 203, 205 du cône 202 situés de part et d'autre de la fente 282. Sur les figures annexées, les zones d'articulation des languettes 260, 270 sur les segments 203, 205 du cône 202 sont référencées 261, 271.

Le sommet des deux segments 203, 205 pourra être arrondi pour répartir l'effort de flexion et ainsi éviter la rupture du matériau choisi.

Plus précisément, comme on l'a illustré en variante sur la figure 6, les zones de transition référencées 201 entre les surfaces externes 204, 206 des segments 203, 205 et les surfaces en regard 264 et 274 des languettes 260 et 270 est de préférence arrondi et non pas anguleux à angle vif pour éviter une rupture de matériau à ce niveau.

Ces zones d'articulation 261, 271 forment des axes d'articulation moyens qui s'étendent perpendiculairement au plan A-A.

Les surfaces axialement extérieures 262, 272 des languettes 260, 270, situées à l'opposé de l'élément de préhension 100, au repos, s'éloignent de l'élément de préhension 100 radialement vers l'extérieur.

Ces surfaces 262, 272 sont de préférence planes.

En d'autres termes, les surfaces 262, 272 des languettes 260, 270 destinées à venir en contact avec la peau du patient, forment un dièdre concave en direction de la peau, lorsque le dispositif est présenté en regard de l'épiderme, l'ensemble d'incision 20 étant dirigé vers la peau.

L'angle α2 formé entre les surfaces 262, 272 et le plan de symétrie B-B est typiquement de 70 à 88°. L'angle est étudié de tel sorte que les extrémités des surfaces 262, 272 entrent en contact avec la peau, après que les extrémités des pointes aient pénétré la surface de l'épiderme, typiquement après que le poinçon 210 ait pénétré d'un tiers environ dans l'épiderme du patient.

A cette fin typiquement l'extrémité 211 du poinçon 210 dépasse d'environ les deux tiers de la hauteur axiale H du poinçon, par rapport au plan radial C-C contenant les bords radialement extérieurs des languettes 260, 270 les plus éloignés axialement de l'élément de préhension 100.

Le contour radialement extérieur 263, 273 des languettes 260, 270 est de préférence arrondi comme on le voit sur les figures.

Les surfaces axialement internes 264, 274 des languettes 260, 270, situées vers l'élément de préhension 100, sont de préférence planes et au repos, s'étendent perpendiculairement au plan B-B.

Il est ainsi formé entre ces surfaces axialement internes 264, 274 des languettes 260, 270, et les surfaces en regard 204, 206 des segments 203, 205 du cône 202, des espaces 208, 209 qui permettent un déplacement à rotation des languettes 260, 270 vers l'élément de préhension 100, lorsque ces languettes 260, 270 sont déplacées de la position de repos vers la position de travail. Ces espaces 208, 209 sont de préférence en forme de dièdre divergeant radialement vers l'extérieur.

Les deux languettes 260, 270 portent par ailleurs, centré sur l'axe A-A le poinçon 210, plus précisément les deux pointes 220, 230 formant le poinçon 210.

Les deux pointes 220, 230 s'étendent axialement vers l'extérieur. Elles sont diamétralement opposées par rapport au plan B-B et sont de préférence venues de matière respectivement sur la partie radialement interne des surfaces axialement externes 262, 272 des languettes 260, 270 destinées à venir en contact avec la peau du patient.

Les pointes 220, 230 sont individualisées par le prolongement de la fente 282 qui débouche axialement sur l'extérieur du dispositif. Au repos leurs extrémités sont cependant adjacentes et se rejoignent au niveau de l'axe O-O sous forme d'un pic affûté d'incision 211.

Chacune des pointes 220, 230 a de préférence la forme d'un demi cône et est délimitée de préférence par une surface radialement interne 222, 232 plane et sensiblement parallèle au plan B-B et une surface externe 224, 234 tronconique ou polyédrique. Les surfaces internes 222, 232 forment un dièdre divergent en direction de l'élément de préhension 10. Les surfaces extérieures coniques 224, 234 forment en combinaison un cône centré sur l'axe A-A dont la base est située du côté de l'élément de préhension 100 et la pointe 211 située à l'opposé de cet élément de préhension 100 sur l'extrémité du dispositif.

Comme indiqué précédemment, il est formé au niveau du cône 202 entre les deux languettes 260, 270 et les pointes 220, 230, un réservoir 280 délimité par une fente 282.

De préférence, la fente 282 débouche latéralement sur le cône 202, c'est-à-dire sur chacun des flancs 102, 104 de l'élément de préhension 100 parallèles au plan de symétrie A-A.

La fente 282 délimite une chambre, formant réservoir de produit, qui s'étend longitudinalement selon l'axe O-O. La fente 282 est de préférence délimitée par deux faces 284, 286 parallèles entre elles, parallèles à l'axe O-O et parallèles au plan de symétrie B-B. La chambre 280 est ainsi de préférence de contour parallélépipédique sur sa partie supérieure.

La distance d1 entre les deux faces 284, 286 est de préférence comprise entre 0,02 et 0,8mm, avantageusement entre 0,05 et 0,4mm. La longueur L2 de la chambre 280 formée par la fente 282 dans le cône et les languettes 260, 270, est de préférence comprise entre 2 et 10mm, avantageusement entre 2 et 6mm.

La chambre parallélépipédique 280 délimitée par les faces 284, 286, se prolonge par un canal 288 effilé, délimité par les surfaces 222, 232 précitées et qui converge progressivement en direction de la pointe 211.

A titre d'exemple non limitatif, le volume du réservoir 280 est avantageusement de l'ordre de 0,2µl, voir compris entre 0,2µl et 0,5µl.

Le dispositif d'incision conforme à la présente invention possède par ailleurs de préférence des moyens 290 de verrouillage des languettes 260, 270 et des pointes 220, 230 en position de travail, une fois celle-ci atteinte.

A cette fin, de préférence comme on le voit sur les figures annexées, le dispositif d'incision conforme à l'invention comprend sur l'extérieur du cône 202, deux bras 292, 294 diamétralement opposés par rapport au plan B-B, s'étendant axialement vers l'extérieur du dispositif et munis chacun à leur extrémité d'un crochet ou ergot 293, 295 dirigé radialement vers l'intérieur, c'est-à-dire vers le plan de symétrie B-B.

Les languettes 260, 270 sont munies de structure de crochets ou ergots complémentaires 265, 275, dirigés radialement vers l'extérieur.

Ces structures de crochet 265, 275 sont formées par des décrochements en dépouille sur une zone de transition entre les faces axialement internes 264, 274 et le contour radialement extérieur 263, 273 des languettes 260, 270. Au repos les structures de crochet 265, 275 sont axialement à l'extérieur des crochets 293, 295 prévus sur les bras 292, 294, tout en étant de préférence adjacents à ceux-ci.

Le diamètre radialement externe des crochets 265, 275 prévus sur les languettes 260, 270 est légèrement supérieur au diamètre radialement interne des crochets 293, 295 prévus sur les bras 292, 294.

Ainsi, lors du déplacement des languettes 260, 270 de la position de repos vers la position de travail, les crochets 265, 275 sollicitent les crochets 293, 295 en déplacement radial vers l'extérieur par déformation élastique des bras 292, 294. Une fois que les crochets 293, 295 ont franchi les crochets 265, 275, les crochets 293, 295 reprennent élastiquement leur position initiale et maintiennent les crochets 265, 275 ainsi que par conséquent les languettes 260, 270 et les pointes 220, 230 en position de travail comme illustré sur la figure 6.

La hauteur axiale H des pointes 220, 230, qui correspond à la profondeur d'incision est typiquement comprise entre 0,5 et 5mm.

L'encombrement radial R des languettes 220, 230 qui correspond au cumul de l'étendue radiale des deux languettes 220 et 230 est typiquement compris entre 4 et 15mm. Cet encombrement radial R peut aisément être adapté en fonction de la résistance des zones charnières 261 et 271 pour définir un effort sur la peau acceptable. A cette fin on peut prévoir des languettes de dimension radiale plus importante, perpendiculairement au plan B-B.

Pour utiliser le système conforme à la présente invention on procède comme suit.

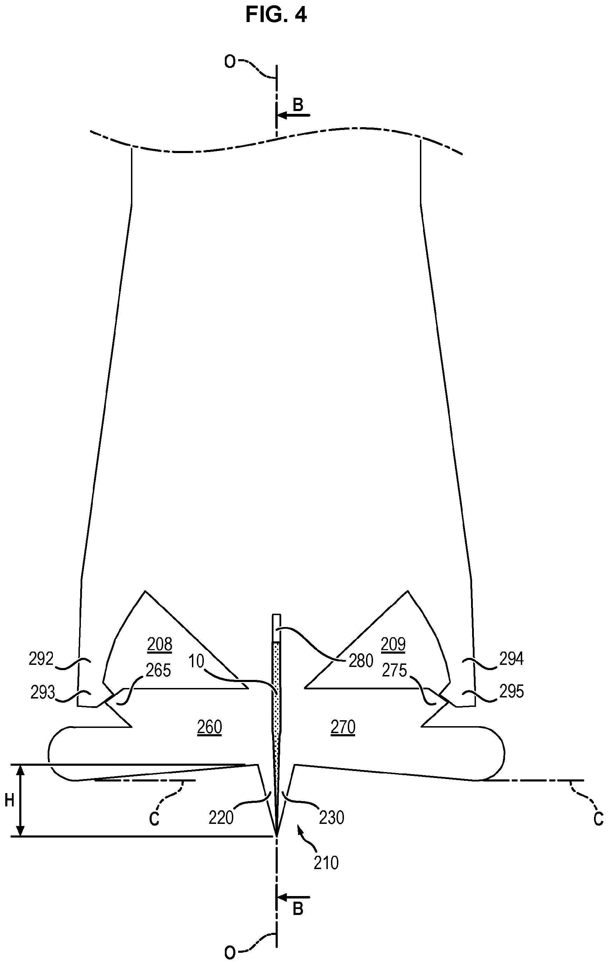

Le réservoir 280 du dispositif doit dans un premier temps être rempli avec du produit adapté pour l'utilisation, par exemple un extrait allergénique, comme cela est illustré sur la figure 4.

Sur les figures annexées le produit est référencé schématiquement 10 et la peau P.

Le réservoir 280 peut être pré-rempli.

Sinon l'extrémité du dispositif doit être porté au contact d'une réserve de produit stocké dans un récipient adéquat pour remplir le réservoir 280 par capillarité.

Le dispositif est alors prêt à être utilisé.

Dans cet état le canal formé entre les surfaces 222 et 232 des deux pointes 220 et 230, est également rempli de produit par capillarité.

Contrairement aux dispositifs classiques, grâce à l'invention, le produit 10 n'est pas déposé sur la peau P à l'aide d'un distributeur. Le poinçon 210 du dispositif est directement trempé dans la réserve de produit et sa conformation fait pénétrer le produit par capillarité dans l'espace 280 en forme de fente, aménagé au sein de l'extrémité du dispositif.

Après avoir repéré la zone d'incision et pris les précautions aseptiques d'usage, le dispositif conforme à la présente invention est porté au contact de la zone choisie du patient, en plaçant l'extrémité du poinçon 210, puis les surfaces 262 et 272 des languettes 260 et 270, au contact de la peau.

Le poinçon 210 pique et pénètre alors la peau. Après enfoncement du poinçon 210, par exemple d'un tiers environ, les languettes 260 et 270 viennent en contact avec la peau. Le contact progressif entre les surfaces 262 et 272 des languettes 260 et 270 et la résistance de la peau provoque le pivotement progressif des languettes 260, 270 et donc l'écartement des pointes 220, 230 comme on l'a schématisé sur la figure 5 pour libérer le produit 10.

Les pointes 220 et 230 ont totalement pénétrées la peau lorsque les surfaces 262 et 272 des languettes 260 et 270 sont complètement accolées à la peau comme illustré sur la figure 6.

Comme on le voit sur cette figure 6, le pivotement des languettes 260 et 270 induit un écartement des surfaces radialement internes en regard 222, 232 des pointes 220, 230 et des surfaces radialement internes en regard 284, 286 délimitant la chambre 280 au-delà de l'articulation. Cet écartement permet une libération du produit contenu dans la chambre 280 et sa pénétration dans l'incision formée dans la peau comme on le voit sur la figure 6 au moment où les deux pointes 220, 230 s'écartent par l'appui des languettes 260, 270 sur la peau.

Les crochets ou ergots complémentaires 265 et 293, respectivement 275 et 295, empêchent alors les pointes ou aiguilles 220 et 230 de revenir à leur position initiale et empêchent ainsi le liquide 10 de remonter par capillarité le long du petit canal formé entre les pointes 220, 230, afin de bien remplir l'espace créé par l'écartement des aiguilles 220, 230.

En position de travail la distance séparant l'extrémité des pointes 220 et 230 est typiquement comprise entre 0,2 et 3mm.

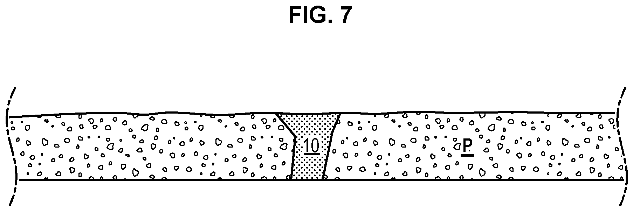

La figure 7 montre une micro-goutte de produit ainsi diffusé dans la partie supérieure de l'épiderme après utilisation du dispositif conforme à la présente invention.

La suite du protocole pour diagnostic (délai d'observation et références pour diagnostic) est classique.

L'on observera que la structure de dispositif conforme à l'invention permet d'optimiser le confort du patient car en cas de pression un peu trop forte, la butée suffisamment large formée par les surfaces 262 et 272 des languettes permet de ne pas faire saigner le patient. Par ailleurs, cette même butée laisse à l'opérateur la possibilité d'une pression un peu plus élevée afin d'éviter les faux négatifs ce qui permet une meilleure fiabilité.

De plus l'injection de produit 10 par ouverture des pointes 220, 230, une fois ces pointes insérées dans la peau P permet une meilleure pénétration du produit au sein de l'épiderme que les moyens connus de l'état de la technique demandant de piquer à travers une goutte préalablement déposée sur la peau.

Le système d'écartement des deux pointes 220, 230 lors de l'utilisation, suivi du maintien en position écartée grâce aux crochets complémentaires 265 et 293, respectivement 275 et 295, lors de l'enlèvement du dispositif, permet de faire entrer de façon certaine une microgoutte d'extrait allergénique 10 suffisamment importante au sein de l'épiderme et donc de diminuer le risque de faux négatifs.

Le dispositif conforme à la présente invention offre de nombreux avantages par rapport aux dispositifs de l'état de la technique.

Il présente notamment les avantages suivants :
- il offre une totale sécurité pour les patients d'une part par l'absence de risque de contamination entre différents patients, du fait que grâce à l'invention, il n'est plus nécessaire ou risquer de porter une pipette ou distributeur de produit au contact de la peau du patient à tester pour déposer une goutte de produit devant être injecté (laquelle pipette est ensuite remise sur le flacon formant réserve de produit pour être réutilisée pour des patient suivants) puisque selon l'invention le dispositif peut être prérempli dans son réservoir 280 de la quantité de produit optimale, avant d'être porté au contact de la peau, et d'autre part grâce à une garantie d'incision à la profondeur optimale et seulement à cette profondeur. Une fois les surfaces 262, 272 complètement accolées à la peau, le dispositif conforme à l'invention garantit en effet que les pointes 220 et 230 sont intégralement insérées dans la peau. Mais ces surfaces 262, 272 garantissent également que les pointes 220 et 230 ne pénètrent pas plus profondément dans les tissus du patient.
- il permet de garantir un parfaite reproductibilité de pénétration du produit.
- il permet d'optimiser la consommation de la quantité de produit nécessaire au diagnostic. L'invention permet en particulier de doser précisément la quantité de produit 10 placé dans le réservoir 280 en fonction de la dose requise. L'invention permet ainsi de réduire considérablement la quantité de produit utilisée par rapport à l'état de la technique qui nécessitait de placer sur la peau une goutte de produit largement surdimensionnée par rapport à la quantité à injecter requise. Typiquement la technique classique du prick test passe par le dépôt d'une goutte d'environ 50 microlitres d'extrait sur l'avant-bras des patients. Seule une infime partie du produit est utile au déclenchement de la réaction cutanée et il est possible d'éliminer le surplus pour le confort du patient. Le volume introduit dans le dispositif conforme à l'invention est typiquement d'environ 0,2 microlitres, soit environ 250 fois moins.

- il permet un processus totalement fiable.
- il permet d'améliorer la rapidité de la séance de test.
   La technique classique oblige au dépôt d'une goutte de produit sur la peau, par exemple sur l'avant-bras, pour chacun des extraits testés, généralement de l'ordre d'une quinzaine d'extraits, avant d'effectuer l'incision elle-même. Le dispositif conforme à l'invention nécessite un simple « pré-trempage » de l'extrémité du dispositif dans une solution d'extrait allergénique sans dépôt avant l'incision, ce qui simplifie et écourte la séance.
- il permet un grand confort du patient. Grâce à l'invention en particulier il n'y a plus d'obligation de rester en position inconfortable pendant la révélation des tests. La technique classique d'un test allergique oblige en effet au dépôt d'une goutte sur la peau, par exemple l'avant-bras, des patients avant l'incision, ce qui oblige les patients à maintenir leurs avant-bras, côté interne vers le haut, afin d'éviter l'écoulement de l'extrait. Cette position n'est pas toujours confortable ni facile à tenir, en particulier chez les jeunes enfants. Le dispositif conforme à l'invention ne passant pas par une étape de dépôt d'extrait, le maintien dans cette position n'est pas nécessaire.

Le dispositif conforme à la présente invention peut être réalisé en tout matériau approprié, par exemple en métal, notamment en acier inoxydable, ou en matière plastique.

Bien entendu la présente invention n'est pas limitée au mode de réalisation qui vient d'être décrit, mais s'étend à toute variante de réalisation conforme à son esprit.

On a décrit précédemment une utilisation du dispositif conforme à la présente invention pour des diagnostics allergiques.

L'invention n'est cependant pas limitée à cette application particulière. Elle peut être utilisée dans toute application demandant une injection cutanée, par exemple pour l'injection d'un vaccin, en adaptant en conséquence la longueur H des pointes 220 et 230.

Par ailleurs on a décrit précédemment un dispositif comprenant deux pointes 220 et 230 diamétralement opposées par rapport au plan B-B.

Dans le cadre de l'invention, le dispositif peut cependant comprendre un nombre de pointes supérieur à deux, associées chacune à un moyen de sollicitation conforme aux languettes 260, 270, équiréparties autour de l'axe O-O.

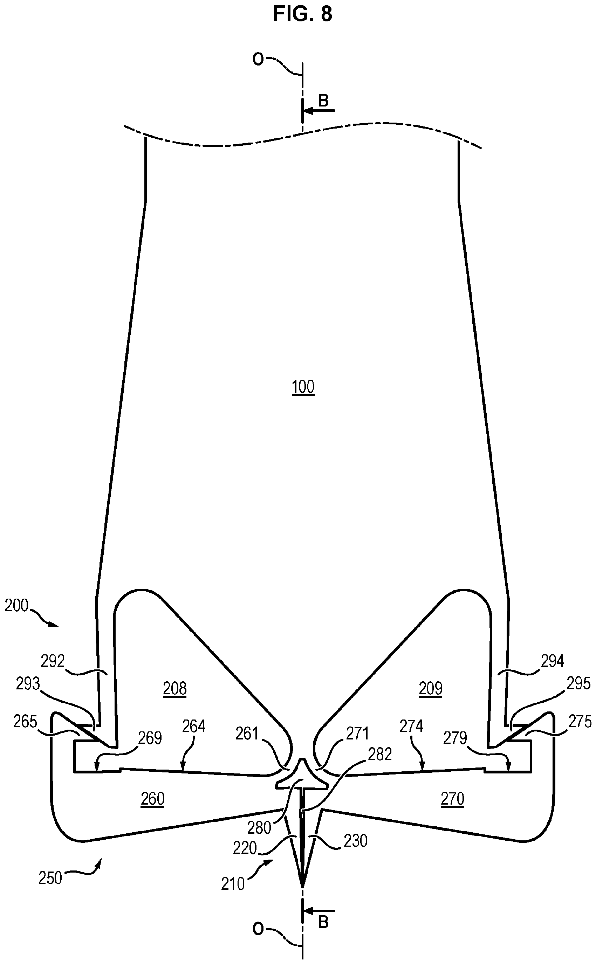

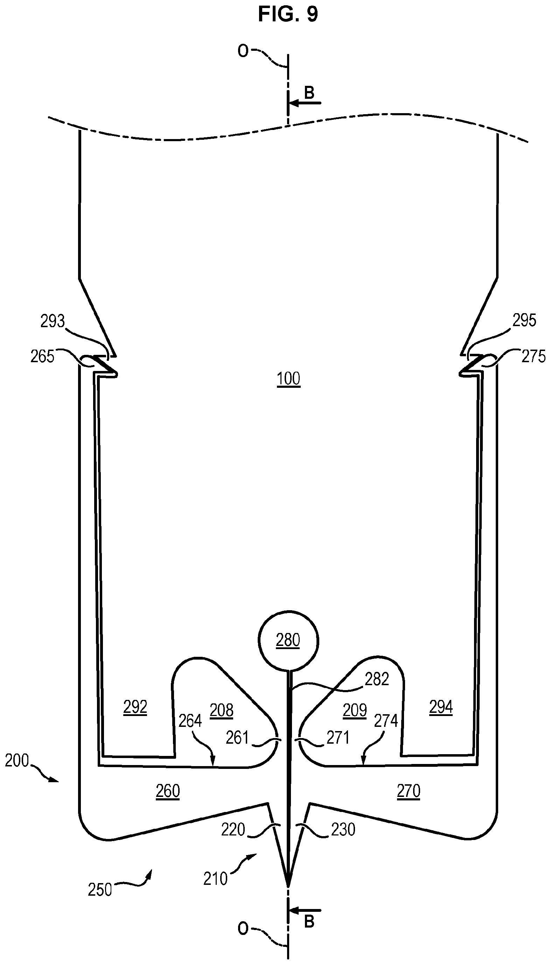

On va maintenant décrire les variantes de réalisation conformes à l'invention illustrées sur les figures 8 et 9.

Sur ces figures 8 et 9 les éléments similaires à ceux précédemment décrits sur les figures 1 à 7 portent des références numériques identiques.

La variante de réalisation illustrée sur la figure 8 se distingue essentiellement du mode de réalisation illustré sur les figures 1 à 7 par les dispositions suivantes :
- les zones charnières 261, 271 sont plus fines et plus arrondies,
- la chambre 280 formant réservoir de produit, réalisée à l'extrémité de la fente 282 est constituée d'une cavité évasée par rapport à la fente, c'est à dire dont les dimensions perpendiculaires au plan B-B sont supérieures à la largeur de la fente 282 considérée perpendiculairement à ce plan B-B,
- l'orientation des crochets 265, 275 et 293, 295 est inversée, c'est-à-dire que selon la figure 8 les structures de crochet 265 et 275 formées sur les languettes 260, 270 sont orientées radialement vers l'intérieur et inversement les structures de crochet 293 et 295 formées sur les bras 292, 294 sont orientées radialement vers l'extérieur,
- les faces axialement internes 264, 274 des languettes 260, 270 possèdent des décrochements 269, 279 placés en regard des extrémités des bras 292, 294 destinés à servir de butée aux bras 292, 294 limitant le risque de fléchissement de ces bras 292, 294 par rapprochement de l'axe O-O.

La variante de réalisation illustrée sur la figure 9 se distingue essentiellement du mode de réalisation illustré sur les figures 1 à 7 par les dispositions suivantes :
- les bras 292 et 294 possèdent une largeur plus importante de sorte que les bras 292 et 294 représentés sur la figure 9 constituent une butée plus forte que les bras représentés sur les figures 1 à 8 et permettent de limiter l'amplitude de déformation des languettes 260, 270 en cas de forte pression,
- les crochets 265/293 et 275/295 servant à maintenir les pointes d'incision 220, 230 en position ouverte sont déplacés à distance des pointes d'incision afin de préserver une meilleure rigidité de l'ensemble d'incision. Cette disposition permet également d'amplifier le signal sonore généré lorsque les crochets 265/293 et 275/295 sont portés en prise.

## Revendications

1. Dispositif d'incision comprenant :
- un élément de préhension manuelle (100) et
- un ensemble d'incision (200) placé sur une extrémité de l'élément de préhension (100) et comprenant un poinçon (210), scindé en au moins deux pointes d'incision (220, 230),
**caractérisé en ce que** l'une au moins de ces pointes (220, 230) est articulée à déplacement sur l'élément de préhension (100) pour être mobile entre une position de repos et d'incision dans laquelle les pointes (220, 230) sont adjacentes et une position de travail et d'injection dans laquelle les pointes (220, 230) sont écartées et que le dispositif comprend des moyens de sollicitation (250) adaptés pour déplacer chaque pointe mobile (220, 230) en position de travail, lorsque ces moyens de sollicitation (250) viennent en appui sur la peau.

2. Dispositif selon la revendication 1, **caractérisé en ce que** le poinçon (210) est scindé en deux pointes (220, 230) d'incision, articulée chacune à rotation sur l'élément de préhension (100) pour être mobile entre une position de repos et d'incision adjacentes et une position de travail et d'injection écartées.

3. Dispositif selon l'une des revendications 1 ou 2, **caractérisé en ce que** chaque pointe articulée à déplacement (220, 230) est reliée à l'élément de préhension (100) par une zone charnière venue de matière.

4. Dispositif selon l'une des revendications 1 à 3, **caractérisé en ce que** les moyens de sollicitation (250) comprennent une languette (260, 270) associée respectivement à chaque pointe (220, 230), qui s'étend perpendiculairement à l'axe longitudinal (O-O) des pointes et qui porte une pointe respective (220, 230), les surfaces axialement extérieures (262, 272) des languettes (260, 270) formant au repos un dièdre concave.

5. Dispositif selon l'une des revendications 1 à 4, **caractérisé en ce qu'**il comporte une chambre (280) formant réserve de produit à injecter (10).

6. Dispositif selon la revendication 5, **caractérisé en ce que** la chambre (280) est formée d'une fente formée dans l'extrémité de l'ensemble de préhension (100) et qui se prolonge dans le poinçon (210) pour individualiser les pointes (230).

7. Dispositif selon l'une des revendications 5 ou 6, **caractérisé en ce que** la chambre (280) formant réserve de produit est constituée d'une cavité évasée formée à l'extrémité d'une fente (282) réalisée dans l'extrémité de l'ensemble de préhension (100) et qui se prolonge dans le poinçon (210) pour individualiser les pointes (230).

8. Dispositif selon l'une des revendications 1 à 7, **caractérisé en ce qu'**il comprend des moyens (290) de verrouillage des pointes (220, 230) en position de travail et d'injection.

9. Dispositif selon la revendication 8, **caractérisé en ce que** les moyens de verrouillage (290) comprennent des crochets complémentaires (265, 275 ; 293, 295) sur les moyens de sollicitation (250) et sur des bras (292, 294) prévus sur l'extrémité de l'ensemble de préhension.

10. Dispositif selon l'une des revendications 8 ou 9, **caractérisé en ce que** les moyens de verrouillage (290) sont placés à distance des pointes d'incision (220, 230) afin de préserver la rigidité de l'ensemble d'incision et induire un signal sonore lorsque les moyens de verrouillage (290) sont portés en prise.

11. Dispositif selon l'une des revendications 1 à 10, **caractérisé en ce que** les pointes (220, 230) ont une hauteur (H) comprise entre 0,5 et 5mm.

12. Dispositif selon l'une des revendications 1 à 11, **caractérisé en ce qu'**il présente un axe longitudinal (O-O) et deux plans de symétrie (A-A, B-B).

13. Dispositif selon l'une des revendications 1 à 12, **caractérisé en ce qu'**il comprend des moyens (292, 294) adaptés pour limiter l'amplitude de déplacement des moyens de sollicitation (250).

14. Dispositif selon la revendication 13, **caractérisé en ce que** les moyens (292, 294) adaptés pour limiter l'amplitude de déplacement des moyens de sollicitation (250) sont formés par des bras (292, 294) liés à l'élément de préhension manuelle (100) et placés en regard des moyens de sollicitation (250) pour leur servir de butée.

## Patentansprüche

1. Einschnittvorrichtung, umfassend:
- ein manuelles Greifelement (100); und
- eine Einschnittgruppe (200), die auf einem Ende des Greifelements (100) angeordnet ist und eine Punkturnadel (210) umfasst, die in wenigstens zwei Einschnittpunkte (220, 230) aufgespalten ist,
**dadurch gekennzeichnet, dass** wenigstens eine dieser Spitzen (220, 230) in Verschiebung auf dem Greifelement (100) artikuliert ist, um zwischen einer Ruhe- und Einschnittposition, in der die Spitzen (220, 230) angrenzend sind, und einer Arbeits- und Einschnittposition, in der die Spitzen (220, 230) beabstandet sind, mobil zu sein, und die Vorrichtung Ansprechmittel (250) umfasst, die geeignet sind, um jeden mobilen Punkt (220, 230) in der Arbeitsposition zu verschieben, wenn diese Ansprechmittel (250) auf der Haut zum Aufstützen kommen.

2. Vorrichtung gemäß Anspruch 1, **dadurch gekennzeichnet, dass** die Punkturnadel (210) in zwei Einschnittpunkte (220, 230) aufgespalten ist, jede in Rotation auf dem Greifelement (100) artikuliert ist, um zwischen einer anliegenden Ruhe- und Einschnittposition und einer beabstandeten Arbeits- und Einspritzposition mobil zu sein.

3. Vorrichtung gemäß einem der Ansprüche 1 oder 2, **dadurch gekennzeichnet, dass** jede artikulierte Spitze mit Verschiebung (220, 230) am Greifelement (100) durch einen Scharnierbereich aus demselben Material verbunden ist.

4. Vorrichtung gemäß einem der Ansprüche 1 bis 3, **dadurch gekennzeichnet, dass** die Ansprechmittel (250) eine Zunge (260, 270) umfassen, die jeweils jeder Spitze (220, 230) zugeordnet ist, die sich lotrecht zur Längsachse (O-O) der Punkte erstreckt und die eine jeweilige Spitze (220, 230) trägt, wobei die axial von den Zungen (260, 270) externen Oberflächen (262, 272) in Ruhe ein konkaves Dieder bilden.

5. Vorrichtung gemäß einem der Ansprüche 1 bis 4, **dadurch gekennzeichnet, dass** sie eine Kammer (280) umfasst, die eine Reserve für das einzuspritzende Produkt (10) bildet.

6. Vorrichtung gemäß Anspruch 5, **dadurch gekennzeichnet, dass** die Kammer (280) aus einem Schlitz gebildet ist, der im Ende der Greifgruppe (100) gebildet ist und sich in der Punkturnadel (210) verlängert, um die Spitzen (230) zu individualisieren.

7. Vorrichtung gemäß einem der Ansprüche 5 oder 6, **dadurch gekennzeichnet, dass** die Kammer (280), die die Produktreserve bildet, aus einer aufgeweiteten Ausnehmung gebildet ist, die am Ende eines Schlitzes (282) gebildet ist, der in dem Ende der Greifgruppe (100) realisiert ist und der sich in der Punkturnadel (210) verlängert, um die Spitzen (230) zu individualisieren.

8. Vorrichtung gemäß einem der Ansprüche 1 bis 7, **dadurch gekennzeichnet, dass** sie Verriegelungsmittel (290) der Spitzen (220, 230) in der Arbeits- und Einspritzposition umfasst.

9. Vorrichtung gemäß Anspruch 8, **dadurch gekennzeichnet, dass** die Verriegelungsmittel (290) komplementäre Haken (265, 275; 293, 295) auf den Ansprechmitteln (250) und auf den Armen (292, 294) umfassen, die auf dem Ende der Greifgruppe vorgesehen sind.

10. Vorrichtung gemäß einem der Ansprüche 8 oder 9, **dadurch gekennzeichnet, dass** die Verriegelungsmittel (290) von den Einschnittspitzen (220, 230) entfernt angeordnet sind, um die Steifigkeit der Einschnittgruppe zu bewahren und ein Tonsignal zu induzieren, wenn die Verriegelungsmittel (290) eingegriffen getragen werden.

11. Vorrichtung gemäß einem der Ansprüche 1 bis 10, **dadurch gekennzeichnet, dass** die Spitzen (220, 230) eine zwischen 0,5 und 5 mm inbegriffene Höhe (H) aufweisen.

12. Vorrichtung gemäß einem der Ansprüche 1 bis 11, **dadurch gekennzeichnet, dass** sie eine Längsachse (O-O) und zwei Symmetrieachsen (A-A, B-B) aufweist.

13. Vorrichtung gemäß einem der Ansprüche 1 bis 12, **dadurch gekennzeichnet, dass** sie Mittel (29, 294) umfasst, die geeignet sind, um die Verschiebungsamplitude der Ansprechmittel (250) zu begrenzen.

14. Verfahren gemäß Anspruch 13, **dadurch gekennzeichnet, dass** die Mittel (292, 294) geeignet sind, um die Verschiebungsamplitude der Ansprechmittel (250) von Armen (292, 294) zu begrenzen, die gebildet sind, die mit dem manuellen Greifmittel (100) verbunden und gegenüber Ansprechmitteln (250) angeordnet sind, um ihnen als Anschlag zu dienen.

## Claims

1. An incision device comprising:
- a manual gripping element (100) and
- an incision assembly (200) placed on an end of the gripping element (100) and comprising a punch (210), split into at least two incision tips (220, 230),
**characterised in that** at least one of these tips (220, 230) is movably hinged to the gripping element (100) to be movable between a rest and incision position in which the tips (220, 230) are adjacent and a working and injection position in which the tips (220, 230) are distant and **in that** the device comprises loading means (250) adapted to move each movable tip (220, 230) into working position, when these loading means (250) bear against the skin.

2. The device according to claim 1, **characterised in that** the punch (210) is split into two incision tips (220, 230), each being rotatably hinged to the gripping element (100) to be movable between an adjacent rest and incision position and a distant working and injection position.

3. The device according to one of claims 1 or 2, **characterised in that** each movably hinged tip (220, 230) is connected to the gripping element (100) by an integral hinge zone.

4. The device according to one of claims 1 to 3, **characterised in that** the loading means (250) comprise a tab (260, 270) respectively associated with each tip (220, 230), which extends perpendicular to the longitudinal axis (O-O) of the tips and which carries a respective tip (220, 230), the axially external surfaces (262, 272) of the tabs (260, 270) forming a concave dihedron at rest.

5. The device according to one of claims 1 to 4, **characterised in that** it includes a chamber (280) forming a stock of product to be injected (10).

6. The device according to claim 5, **characterised in that** the chamber (280) is formed by a slit formed in the end of the gripping assembly (100) and which extends in the punch (210) to individualise the tips (230).

7. The device according to one of claims 5 or 6, **characterised in that** the chamber (280) forming a stock of product consists of a flared cavity formed at the end of a slit (282) made in the end of the gripping assembly (100) and which extends in the punch (210) to individualise the tips (230).

8. The device according to one of claims 1 to 7, **characterised in that** it comprises means (290) for locking the tips (220, 230) into working and injection position.

9. The device according to claim 8, **characterised in that** the locking means (290) comprise complementary hooks (265, 275; 293, 295) on the loading means (250) and on arms (292, 294) provided on the end of the gripping assembly.

10. The device according to one of claims 8 or 9, **characterised in that** the locking means (290) are placed at a distance from the incision tips (220, 230) in order to keep the rigidity of the incision assembly and induce a sound signal when the locking means (290) are brought into engagement.

11. The device according to one of claims 1 to 10, **characterised in that** the tips (220, 230) have a height (H) between 0.5 and 5mm.

12. The device according to one of claims 1 to 11, **characterised in that** it has one longitudinal axis (O-O) and two planes of symmetry (A-A, B-B).

13. The device according to one of claims 1 to 12, **characterised in that** it comprises means (292, 294) adapted to limit movement amplitude of the loading means (250).

14. The device according to claim 13, **characterised in that** the means (292, 294) adapted to limit movement amplitude of the loading means (250) are formed by arms (292, 294) linked to the manual gripping element (100) and placed facing the loading means (250) to act as abutment therefor.
